# EUROPEAN PATENT SPECIFICATION

(11) **EP 3 421 089 B1**
(45) Date of publication and mention of the grant of the patent: **13.05.2020**
(21) Application number: 17785276.1
(22) Date of filing: 16.03.2017
(51) Int. Cl.: A61K 31/69, A61N 5/10, A61P 25/28, C07D 207/00, C07D 207/02, C07D 207/04

(54) **NEUTRON CAPTURE THERAPY SYSTEM FOR ELIMINATING AMYLOID -PROTEIN PLAQUE**
NEUTRONENEINFANGTHERAPIESYSTEM ZUR BESEITIGUNG VON AMYLOID-PROTEIN-PLAQUE
SYSTÈME DE THÉRAPIE PAR CAPTURE DE NEUTRONS POUR ÉLIMINER UNE PLAQUE DE PROTÉINE BÊTA-AMYLOÏDE

(30) Priority: 19.04.2016 CN 201610242672
(43) Date of publication of application: 02.01.2019
(73) Proprietor: Neuboron Medtech Ltd., Nanjing, Jiangsu 211112 (CN)
(72) Inventor: CHEN, Jui-fen, Nanjing Jiangsu 211112 (CN); HE, Jing, Nanjing Jiangsu 211112 (CN); LIU, Yuan-hao, Nanjing Jiangsu 211112 (CN)
(74) Representative: Sun, Yiming
(86) International application number: PCT/CN2017/076935
(87) International publication number: WO 2017/181790

(56) References cited:
- CN-A- 101 668 547
- CN-A- 106 552 321
- CN-A- 106 552 322
- CN-U- 205 073 541
- CN-U- 205 073 542
- Ryu Tada ET AL: "An NDT study of a boron tracedrug UTX-51 for glycated BSA as an AGE model", Anticancer research, 1 August 2014 (2014-08-01), page 4503, XP055546811, Greece Retrieved from the Internet: URL:http://ar.iiarjournals.org/content/34/ 8/4503.full.pdf [retrieved on 2019-01-23]
- Protti: "Innovative therapies to treat Alzheimer's Disease: an interview with Nicoletta Protti", , 1 January 2015 (2015-01-01), XP055546537, Retrieved from the Internet: URL:https://www.researchitaly.it/en/interv iews/innovative-therapies-to-treat-alzheim er-s-disease-an-interview-with-nicoletta-p rotti/ [retrieved on 2019-01-23]
- JUNG, S.J. et al.: "Discovery of Boronic Acid-based Fluorescent Probes Targeting Amyloid-beta Plaques in Alzheimer's Disease", BIOORGANIC & MEDICINAL CHEMISTRY LETTERS, vol. 26, 16 February 2016 (2016-02-16), pages 1784-1788, XP029453943,

## Description

### FIELD OF THE DISCLOSURE

The present disclosure relates to a neutron capture therapy system, in particular to a neutron capture therapy system that can be used to eliminate amyloid β-protein.

### BACKGROUND OF THE DISCLOSURE

Alzheimer's disease (AD) is a latent, progressive, and irreversible brain disease with a high incidence among people over the age of 65. The current therapeutic goal of AD is to maintain physical function and ability while slowing or delaying symptoms. Treatments for mild-to-moderate AD include acetylcholinesterase inhibitors such as Donepezil, Rivastigmine, and Galantamine. Donepezil is also used for the treatment of moderate to severe AD, alone or in combination with the N-methyl-D-aspartate receptor antagonist Memantine. These neurotransmitter-modulating drugs may temporarily improve symptoms, but patients still experience progressive deterioration in cognitive abilities, as well as mental illness, restlessness, depression, and sleep disturbances.

In 1984, for the first time, scientists purified and determined the amino acid sequence of Aβ from the meningeal blood vessel wall of AD patients. Their basic structure contains peptides of 40 or 42 amino acids, collectively referred to as amyloid β-protein. In human cerebrospinal fluid and plasma, the level of Aβ₁₋₄₀ is 10-fold and 1.5-fold higher than that of Aβ₁₋₄₂, respectively. Aβ₁₋₄₂ is more toxic and more likely to accumulate, forming the core of Aβ precipitation and triggering neurotoxicity. The Aβ cascade hypothesis suggests that in AD patients due to deposition of excessive Aβ or Aβ₁₋₄₂ with high accumulation ability produced by mutations in the APP and PS genes, it may be toxic to surrounding synapses and neurons, and ultimately cause neuronal cell death. Because abnormal secretion and excessive production of Aβ will lead to other pathological changes in AD, it is the key of the pathogenesis of AD.

At present, the main focus of the development of new drugs for AD treatment is the inhibition of Aβ aggregation and elimination of Aβ. Gantenerumab is a monoclonal antibody that binds to the N-terminal epitope of Aβ. Gantenerumab binds oligomeric and fibrous Aβ, resulting in microglia-mediated elimination of plaque by phagocytes. A previous phase III clinical trial for patients with mild-to-moderate AD has failed, and a phase III clinical trial is currently ongoing for patients with early-stage AD. The latest results showed that Gantenerumab significantly reduces tau protein level in cerebrospinal fluid, but does not significantly reduce Aβ level in cerebrospinal fluid.

Aducanumab is a monoclonal antibody that targets only Aβ in aggregated form. Despite the poor ability of the antibody to cross the blood-brain barrier, Aducanumab can accumulate in the brain due to its significantly elongated half-life in plasma. Early data from Phase lb trial showed that Aducanumab significantly reduced Aβ deposition. The latest PRIME data published at the 2015 Alzheimer's Association International Conference showed that one-year treatment with Aducanumab did not significantly reduce cognitive decline, and the side effects were relatively high.

Reported was the use of the boron tracedrug UTX-51, under thermal neutron irradiation, as an NDT for the targeted clearance of glycated bovine serum albumin as a model for advanced glycation end-products (AGEs) (see Tada et al., Anticancer Research 34: 4503-4508 (2014)). It was shown that NDT with boron tracedrugs can be used for the treatment of AGEs-related diseases.

Neutron capture therapy is a therapeutic technique that is well-targeted, effective, and less harmful to normal tissues. At present, there is no method that can effectively reduce or eliminate amyloid β-protein deposition plaque, and no studies have reported the application of neutron capture therapy to the treatment of Alzheimer's disease.

### SUMMARY

In order to effectively reduce or eliminate amyloid β-protein deposition plaque, the present disclosure provides a neutron capture therapy system for eliminating amyloid β-protein deposition plaque, which comprises a neutron capture therapy device and a ¹⁰B-containing compound, wherein the ¹⁰B-containing compound is capable of specifically binding to the amyloid β-protein deposition plaque, and the energy generated by action of a neutron beam generated by the neutron capture therapy device on the ¹⁰B-containing compound destroys the amyloid β-protein deposition plaque that is specifically bound to the ¹⁰B-containing compound.

Element ¹⁰B has a large capture cross section for thermal neutrons and the essential constituent elements of human body C, H, O, N, P and S have a small capture cross section for thermal neutrons. The ¹⁰B-containing compound is irradiated with thermal neutrons and the reactions shown in Reaction Formula I, and the energy generated by the reactions destroys the substance that specifically binds to the ¹⁰B-containing compound.

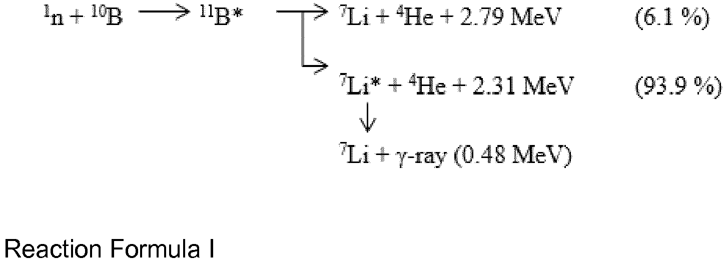

According to this property, when a person taking a ¹⁰B-containing compound is subjected to neutron beam irradiation, the epithermal neutron beam is slowed into thermal neutrons through body tissues and absorbed by the ¹⁰B-containing compound, while there is no damage to the tissue containing no ¹⁰B-containing compound. Since the ¹⁰B-containing compound is capable of specifically binding to amyloid β-protein deposition plaque, when irradiated with a neutron beam, the energy generated by the thermal neutrons and the ¹⁰B-containing compound destroys the structure of amyloid β-protein deposition plaque surrounding the ¹⁰B-containing compound to reduce or eliminate amyloid β-protein deposition plaque.

The present invention provides a neutron capture therapy system for eliminating amyloid β-protein deposition plaque, comprising:
a neutron capture therapy device, and
a ¹⁰B-containing compound having a structure shown in structural formula I: 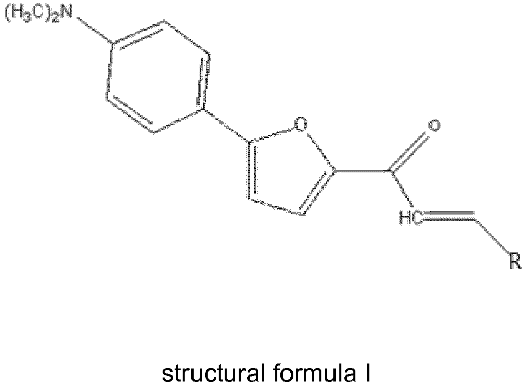
   wherein, R is a phenylboronic acid group, and the boron in the phenylboronic acid group is ¹⁰B;
   wherein the ¹⁰B-containing compound is capable of specifically binding to the amyloid β-protein deposition plaque, and the energy generated by action of a neutron beam generated by the neutron capture therapy device on the ¹⁰B-containing compound destroys the amyloid β-protein deposition plaque that is specifically bound to the ¹⁰B-containing compound.

Preferably, in the neutron capture therapy system for eliminating amyloid β-protein deposition plaque, the neutron capture therapy device comprises a neutron source, a beam shaping assembly, and a collimator, wherein the neutron source is used to generate a neutron beam, the beam shaping assembly is located at the rear of the neutron source and adjusts fast neutrons in the neutron beam having a wide energy spectrum generated by the neutron source to epithermal neutrons, and the collimator is used to converge the epithermal neutrons.

Preferably, in the neutron capture therapy system for eliminating amyloid β-protein deposition plaque, the neutron source comprises an accelerator neutron source or a reactor neutron source.

Preferably, in the neutron capture therapy system for eliminating amyloid β-protein deposition plaque, the beam shaping assembly includes a reflector, a moderator, a thermal neutron absorber, and a radiation shield, wherein the reflector surrounds the moderator for reflecting neutrons diffused towards outside of the beam shaping assembly back into the moderator, the moderator is used to slow fast neutrons into epithermal neutrons, the thermal neutron absorber is used to absorb thermal neutrons to avoid overdosing in superficial normal tissues during therapy, and the radiation shield is used to shield leaked neutrons and photons to reduce the normal tissue dose in non-irradiated areas.

The neutron capture therapy device in the neutron capture therapy system includes a neutron source for generating neutrons and is classified as an accelerator neutron source and a reactor neutron source according to the neutron production principle. The neutron capture therapy device further includes a beam shaping assembly and a collimator. Since the neutron source produces a very wide spectrum of neutrons, these neutrons are classified into fast neutrons, epithermal neutrons, and thermal neutrons according to their energy ranges, wherein the fast neutron energy range is greater than 40keV, the epithermal neutron energy range is between 0.5eV and 40keV, and the thermal neutron energy range is less than 0.5eV. The ¹⁰B-containing compound has a large capture cross section for thermal neutrons, but in actual operation, the neutron beam will be retarded by other substances during the process of reaching the ¹⁰B-containing compound. Therefore, in practical applications, an epithermal neutron beam is often selected to irradiate the ¹⁰B-containing compound. The beam shaping assembly further includes a reflector and a moderator, wherein the moderator is used to slow down the fast neutrons generated by the neutron source into neutrons in the epithermal neutron energy range. The material of the moderator may be composed of one or several combinations of Al₂O₃, BaF₂, CaF₂, CF₂, PbF₂, PbF₄ and D₂O, or the aforesaid material added with lithium-containing material, such as ⁶Li-containing LiF or ⁶Li-containing Li₂CO₃. The reflector is located surrounding the moderator and is generally made of a material having strong neutron reflection ability, such as at least one of Pb-containing material or Ni-containing material. The function of the reflector is to reflect neutrons that spread to the periphery, thereby enhancing the intensity of the neutrons beam. The collimator is located at the rear of the moderator and is used to converge the neutron beam to make the treatment more precise.

In the neutron capture therapy system for eliminating amyloid β-protein deposition plaque, the structural formula of the ¹⁰B-containing compound is: 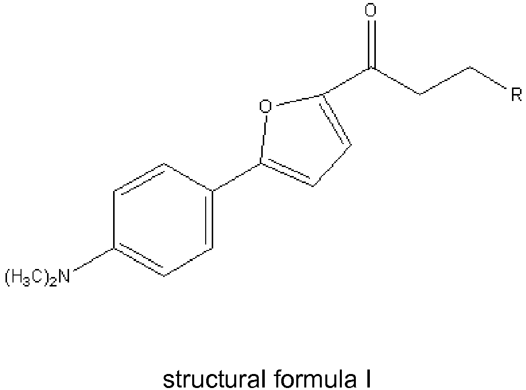 wherein, R is a phenylboronic acid group, and the boron in the phenylboronic acid group is ¹⁰B.

The ¹⁰B-containing compound as shown in structural formula I is capable of specifically binding to amyloid β-protein deposition plaque, and the compound can penetrate the blood-brain barrier.

Preferably, in the neutron capture therapy system for eliminating amyloid β-protein deposition plaque, the R group in the structural formula I comprises R₁ and R₂ according to different substitution positions of the boronicacid group, wherein R₁ group is: 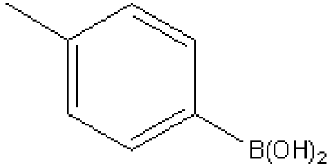 R₂ group is: 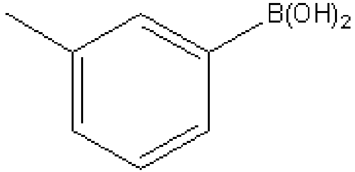 when the substituent R in the ¹⁰B-containing compound is R₁, the ¹⁰B-containing compound is Compound I; when the substituent R in the ¹⁰B-containing compound is R₂, the ¹⁰B-containing compound is Compound II.

Preferably, in the neutron capture therapy system for eliminating amyloid β-protein deposition plaque, the amyloid β-protein deposition plaque includes Aβ₄₂. The amyloid β-protein deposition plaque is mainly formed by high accumulation of Aβ₄₂ which is easy to accumulate. amyloid β-protein deposition plaque can cause neurotoxic effects, leading to decreased cognitive abilities and symptoms of Alzheimer's disease.

In the neutron capture therapy system for eliminating amyloid β-protein deposition plaque provided by the present disclosure, neutrons are generated from the neutron source in the neutron capture therapy device. The beam shaping assembly in the neutron capture therapy device adjusts the neutron beam with a broad energy spectrum to a neutron beam that can be captured by the ¹⁰B element with a large cross section. The collimator in the neutron capture therapy device is used to converge the neutron beam to increase the accuracy of the irradiation. The neutron beam exiting the collimator is irradiated on the ¹⁰B-containing compound that has specifically bound to the amyloid β-protein, and the energy generated by the reaction of the neutron and the ¹⁰B element destroys the amyloid β-protein deposition plaque. The capture cross section of ¹⁰B element to thermal neutrons is one hundred times more than these of the essential elements of human body. In other words, thermal neutrons are specific for the ¹⁰B element, while the ¹⁰B-containing compound can bind specifically to amyloid β-protein deposition plaque. Therefore, the neutron capture therapy system for eliminating amyloid β-protein deposition plaque provided by the present disclosure can effectively reduce or eliminate amyloid β-protein deposition plaque.

### DESCRIPTION OF THE FIGURES

FIG. 1 is a schematic plan view of a neutron capture therapy system for eliminating amyloid β-protein deposition plaque.
FIG. 2 is an SDS-PAGE electrophoretogram of a mixed solution of bovine serum albumin and H₃¹⁰BO₃ irradiated with radiation at different positions from the exit of the collimator.

### DESCRIPTION OF THE DISCLOSURE

The following further describes the present disclosure in detail with reference to the accompanying drawings so that those skilled in the art can implement the present disclosure with reference to the specification text.

It will be understood that terms such as "having," "including," and "comprising" used herein do not exclude the presence or addition of one or more other components or combinations thereof.

Alzheimer's disease is a degenerative disease of the central nervous system characterized by progressive cognitive dysfunction and behavioral impairment, which occurs in the senectitude and presenium. The senile plaque is an important pathological feature of Alzheimer's disease. The main component of senile plaques is amyloid β-protein (Aβ). The current research indicates that Aβ is a pathogenic substance of Alzheimer's disease, and overproduction and deposition of Aβ in the brain can cause neuronal synaptic dysfunction.

Aβ₄₂ in the amyloid β-protein deposition plaque has an ability to highly aggregate. After being produced and secreted by neurons, it rapidly aggregates to form a soluble state of oligomers, which are then further aggregated to form Aβ plaques and are deposited in the brain. The amyloid β-protein deposition plaque is a major cause of axonal degeneration and inflammatory responses. Therefore, how to reduce the amyloid β-protein deposition plaque in the brain becomes an important strategy for preventing or treating Alzheimer's disease.

With the advancement of technology, neutron capture therapy has been widely studied as a treatment method with strong targeting, good therapeutic effect, and less damage to normal tissues. However, the application of this technology has focused on the treatment of cancer. At present, it has not been found that this technique with high accuracy and therapeutic effect is used in the treatment of Alzheimer's disease.

The use of neutron capture therapy as an effective treatment for cancer has increased in recent years, with boron neutron capture therapy being the most common. Neutrons in the boron neutron capture therapy can be supplied by nuclear reactors or accelerators. The embodiments of the present disclosure take the accelerator-based boron neutron capture therapy as an example. The basic components of the accelerator-based boron neutron capture therapy generally include an accelerator for accelerating charged particles (e.g., protons, deuterons, etc.), target and thermal removal system and a beam shaping assembly, wherein accelerated charged particles interact with metal target to produce neutrons. The appropriate nuclear reaction is selected according to the desired neutron yield and energy, the available accelerated particle energy, current magnitude, the physicochemical properties of the metal target and other characteristics, and the commonly discussed nuclear reactions are ⁷Li(p,n)⁷Be and ⁹Be(p,n)⁹B, both of which are endothermic reactions. The energy thresholds of the two nuclear reactions are 1.881 MeV and 2.055 MeV, respectively. Since the ideal source of neutrons for boron neutron capture therapy is keV energy-grade epithermal neutrons, theoretically, if a metal lithium target is bombarded with protons with energy just slightly higher than the threshold, relatively low-energy neutrons can be produced and can be used clinically without too much retarding process. However, the proton interaction cross sections of the two targets lithium metal (Li) and beryllium metal (Be) for protons with threshold energy are not big enough. In order to generate a sufficiently large neutron flux, protons with higher energy are usually chosen to initiate the nuclear reaction.

The target, considered perfect, is supposed to have the advantages of high neutron yield, a produced neutron energy distribution near the epithermal neutron energy range (see details thereinafter), little strong-penetration radiation, safety, low cost, easy accessibility, high temperature resistance etc. But in reality, no nuclear reactions may satisfy all requests. The target in these embodiments of the present disclosure is made of lithium. However, well known by those skilled in the art, the target materials may be made of other metals besides the above-mentioned.

Requirements for the heat removal system differ as the selected nuclear reactions. ⁷Li(p, n)⁷Be asks for more than ⁹Be(p, n)⁹B does because of low melting point and poor thermal conductivity coefficient of the metal (lithium) target. In these embodiments of the present disclosure is ⁷Li(p, n)⁷Be.

No matter BNCT neutron sources are from the nuclear reactor or the nuclear reactions between the accelerator charged particles and the target, only mixed radiation fields are produced, that is, beams comprise neutrons and photons having energies from low to high. As for BNCT in the depth of tumors, except the epithermal neutrons, the more the residual quantity of radiation ray is, the higher the proportion of nonselective dose deposition in the normal tissue is. Therefore, radiation causing unnecessary dose should be lowered down as much as possible. Besides air beam quality factors, dose is calculated using a human head tissue prosthesis in order to understand dose distribution of the neutrons in the human body. The prosthesis beam quality factors are later used as design reference to the neutron beams, which is elaborated hereinafter.

The International Atomic Energy Agency (IAEA) has given five suggestions on the air beam quality factors for the clinical BNCT neutron sources. The suggestions may be used for differentiating the neutron sources and as reference for selecting neutron production pathways and designing the beam shaping assembly, and are shown as follows:
Epithermal neutron flux > 1×10⁹ n/cm²s
Fast neutron contamination < 2×10⁻¹³ Gy-cm²/n
Photon contamination < 2×10⁻¹³ Gy-cm²/n
Thermal to epithermal neutron flux ratio < 0.05
Epithermal neutron current to flux ratio > 0.7

Note: the epithermal neutron energy range is between 0.5eV and 40keV, the thermal neutron energy range is lower than 0.5eV, and the fast neutron energy range is higher than 40keV.

### 1. Epithermal neutron flux

The epithermal neutron flux and the concentration of the boronated pharmaceuticals at the tumor site codetermine clinical therapy time. If the boronated pharmaceuticals at the tumor site are high enough in concentration, the epithermal neutron flux may be reduced. On the contrary, if the concentration of the boronated pharmaceuticals in the tumors is at a low level, it is required that the epithermal neutrons in the high epithermal neutron flux should provide enough doses to the tumors. The given standard on the epithermal neutron flux from IAEA is more than 10⁹ epithermal neutrons per square centimeter per second. In this flux of neutron beams, therapy time may be approximately controlled shorter than an hour with the boronated pharmaceuticals. Thus, except that patients are well positioned and feel more comfortable in shorter therapy time, and limited residence time of the boronated pharmaceuticals in the tumors may be effectively utilized.

### 2. Fast neutron contamination

Unnecessary dose on the normal tissue produced by fast neutrons are considered as contamination. The dose exhibit positive correlation to neutron energy, hence, the quantity of the fast neutrons in the neutron beams should be reduced to the greatest extent. Dose of the fast neutrons per unit epithermal neutron flux is defined as the fast neutron contamination, and according to IAEA, it is supposed to be less than 2*10⁻¹³Gy-cm²/n.

### 3. Photon contamination (gamma-ray contamination)

Gamma-ray long-range penetration radiation will selectively result in dose deposit of all tissues in beam paths, so that lowering the quantity of gamma-ray is also the exclusive requirement in neutron beam design. Gamma-ray dose accompanied per unit epithermal neutron flux is defined as gamma-ray contamination which is suggested being less than 2*10⁻¹³Gy-Cm²/n according to IAEA.

### 4. Thermal to epithermal neutron flux ratio

The thermal neutrons are so fast in rate of decay and poor in penetration that they leave most of energy in skin tissue after entering the body. Except for skin tumors like melanocytoma, the thermal neutrons serve as neutron sources of BNCT, in other cases like brain tumors, the quantity of the thermal neutrons has to be lowered. The thermal to epithermal neutron flux ratio is recommended at lower than 0.05 in accordance with IAEA.

### 5. Epithermal neutron current to flux ratio

The epithermal neutron current to flux ratio stands for beam direction, the higher the ratio is, the better the forward direction of the neutron beams is, and the neutron beams in the better forward direction may reduce dose surrounding the normal tissue resulted from neutron scattering. In addition, treatable depth as well as positioning posture is improved. The epithermal neutron current to flux ratio is better of larger than 0.7 according to IAEA.

The prosthesis beam quality factors are deduced by virtue of the dose distribution in the tissue obtained by the prosthesis according to a dose-depth curve of the normal tissue and the tumors. The three parameters as follows may be used for comparing different neutron beam therapy effects.

### 1. Advantage depth

Tumor dose is equal to the depth of the maximum dose of the normal tissue. Dose of the tumor cells at a position behind the depth is less than the maximum dose of the normal tissue, that is, boron neutron capture loses its advantages. The advantage depth indicates penetrability of neutron beams. Calculated in cm, the larger the advantage depth is, the larger the treatable tumor depth is.

### 2. Advantage depth dose rate

The advantage depth dose rate is the tumor dose rate of the advantage depth and also equal to the maximum dose rate of the normal tissue. It may have effects on length of the therapy time as the total dose on the normal tissue is a factor capable of influencing the total dose given to the tumors. The higher it is, the shorter the irradiation time for giving a certain dose on the tumors is, calculated by cGy/mA-min.

### 3. Advantage ratio

The average dose ratio received by the tumors and the normal tissue from the brain surface to the advantage depth is called as advantage ratio. The average ratio may be calculated using dose-depth curvilinear integral. The higher the advantage ratio is, the better the therapy effect of the neutron beams is.

To provide comparison reference to design of the beam shaping assembly, we also provide the following parameters for evaluating expression advantages and disadvantages of the neutron beams in the embodiments of the present disclosure except the air beam quality factors of IAEA and the abovementioned parameters.
1. Irradiation time ≤30min (proton current for accelerator is 10mA)
2. 30.0RBE-Gy treatable depth≥7cm
3. The maximum tumor dose≥60.0RBE-Gy
4. The maximum dose of normal brain tissue ≤12.5RBE-Gy
5. The maximum skin dose ≤11.0RBE-Gy

Note: RBE stands for relative biological effectiveness. Since photons and neutrons express different biological effectiveness, the dose above should be multiplied with RBE of different tissues to obtain equivalent dose.

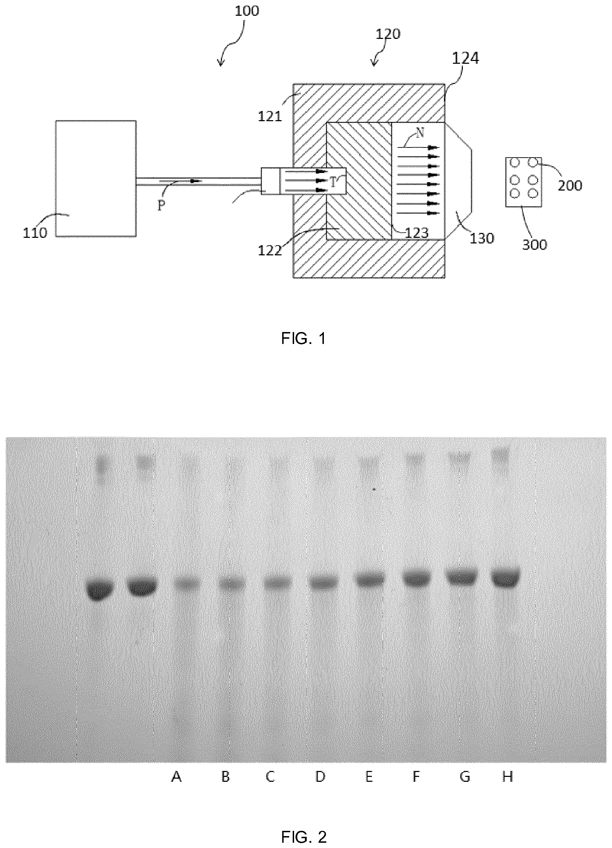

As shown in FIG. 1, the neutron capture therapy system comprises a neutron capture therapy device 100 and a ¹⁰B-containing compound 200, wherein the neutron capture therapy device 100 comprises a neutron source 110, a beam shaping assembly 120, and a collimator 130. The neutron source 110 is classified into an accelerator-type neutron source and a reactor-type neutron source according to the mechanism of neutron generation, and the accelerator-type neutron source is widely used. The accelerator neutron source uses accelerator-accelerated charged particles to bombard an appropriate target nucleus T and generates neutrons through a nuclear reaction. At present, a material commonly used as a target nucleus T is a material containing ⁷Li or ⁹Be.

Regardless of whether the neutron source of the boron neutron capture therapy is from the nuclear reactor or from the nuclear reaction of the charged particles with the target in the accelerator, a mixed radiation field is generated, i.e., the beam contains neutrons and photons from low energy to high energy. These neutrons are classified into fast neutrons, epithermal neutrons, and thermal neutrons based on their energy. Regarding the neutron capture therapy, except for epithermal neutrons, the greater the amount of rest of the radiation is, the greater the proportion of non-selective dose deposition in normal tissue is, so this radiation which will cause unnecessary doses should be minimized. The beam shaping assembly functions to reduce unnecessary doses and enhances the effect of the epithermal neutron beam.

The beam shaping assembly 120 includes a moderator 122, a reflector 121 surrounding the moderator 122, and a thermal neutron absorber 123 adjacent to the moderator 122. The moderator 122 decelerates fast neutrons in the mixed radiation field into the epithermal neutron energy range. The material of the moderator 122 is containing at least one of LiF, Li₂CO₃, Al₂O₃, AlF₃, CaF₂ and MgF₂, wherein the material of the moderator 122 is converted into a block from powder or powder compact in a powder sintering process by a powder sintering apparatus. The reflector 121 redirects neutrons that have been deviated to the periphery to increase the intensity of the epithermal neutron beam. The thermal neutron absorber 123 is used to absorb thermal neutrons to avoid overdosing in superficial normal tissues during therapy. The collimator 130 is located at the rear of the moderator 122 for converging the neutron beam so that the neutron beam has precise directivity during the therapy. The radiation shield 124 is located at the rear of the moderator 122 for shielding the leaked neutrons and photons to reduce the normal tissue dose in the non-irradiated area.

The ¹⁰B-containing compound 200 described in the summary of the disclosure is bound to the amyloid β-protein deposition plaque 300. When the concentration of the ¹⁰B-containing compound on the amyloid β-protein deposition plaque 300 is the highest, it is irradiated with the neutron beam N emitted from the neutron capture therapy device 100, and an appropriate moderator 122 is selected based on the location of the amyloid β-protein deposition plaque 300 so that the energy of the neutron beam is in the epithermal neutron energy range upon reaching the ¹⁰B-containing compound 200 that specifically binds to the amyloid β-protein deposition plaque 300. The epithermal neutron energy range is between 0.5eV and 40keV, and the structure of the amyloid β-protein deposition plaque is destroyed by the energy generated by the reaction of the thermal neutron beam obtained by retarding the epithermal neutron beam with the ¹⁰B element. The ¹⁰B-containing compounds described in the examples of the present disclosure are divided into Compound I and Compound II according to the different substituent R.

The technical solutions of the present disclosure are further described below by way of examples.

### Example 1. Preparation method of ¹⁰B-containing compound

The preparation method of the ¹⁰B-containing compound according to the present disclosure was as follows:
Step 1: 90 mmol of 2-acetylfuran was dissolved in 40mL of dimethylformamide (DMF), then 108mmol of N-bromosuccinimide (NBS) was added at 0°C. The mixture was stirred overnight at room temperature. The mixture contained 1-(5-bromo-2-furyl)ethanone after reaction.

The reaction mixture described in Step 1 was diluted with ethyl acetate and filtered. The organic phase in the filtrate was washed with saturated salt solution and dried over anhydrous sodium sulfate, concentrated, and separated by chromatography to yield 1-(5-bromo-2-furyl)ethanone.
¹H NMR (500 MHz, CDCl₃, δ, ppm): 2.46 (3H. s), 6.49 (1H, d, *J* = 3.4 Hz), 7.12 (1H, d, *J* = 3.4 Hz). MS *m*/*z* 188 (M + H)⁺.

Step 2: To a mixed solution of 10mL of 2M sodium carbonate and 10mL of dimethyl ether (DME), 5.3mmol of 1-(5-bromo-2-furyl)ethanone and 5.3mmol of 4-(dimethylamino)phenylboronic acid were added. Tetrakis(triphenylphosphine)palladium (Pd(PPh₃)₄) was added at 0°C, and the solution reacted at 80°C for 24h. Then the resulting reaction mixture contained 1-(5-(4-dimethylaminobenzene)-2-furyl)ethanone;

Wherein the boron in the 4-(dimethylamino)phenylboronic acid is ¹⁰B.

The reaction mixture described in Step 2 was diluted with ethyl acetate and filtered. The organic phase in the filtrate was washed with saturated salt solution and dried over anhydrous sodium sulfate, concentrated, and separated by chromatography to yield 1-(5-(4-dimethylamino benzene)-2-furyl)ethanone.
¹H NMR (500 MHz, CDCl₃, δ, ppm): 2.49 (3H. s), 3.02 (6H. s), 6.56 (1H, d, *J* = 3.7 Hz), 6.72 (2H, d, *J* = 9.1 Hz), 7.25 (1H, d, *J* = 3.7 Hz), 7.67 (2H, d, *J* = 9.1 Hz). MS *m*/*z* 230 (M + H)⁺.

Step 3: To a dimethylformamide (DMF) solution (50 mL, 1:1) with 2.1 mmol of 1-(5-(4-dimethylaminophenyl)-2-furyl)ethanone and 2.1 mmol of benzaldehyde derivative dissolved therein, NaOH at a concentration of 5M was added at 0°C, and the reaction mixture was stirred at room temperature for 8 h. The resulting mixed solution contained Compound I and Compound II.

The mixed solution containing Compound I and Compound II was adjusted to pH=6 with 1M HCl, filtered, and the solid substance obtained by filtration was separated by chromatography to yield Compound I:
¹H NMR (500 MHz, DMSO-d₆, δ, ppm): 2.96 (6H. s), 6.78 (2H, d, *J* = 8.8 Hz), 6.97 (1H, d, *J* = 3.7 Hz), 7.67-7.71 (4H, m), 7.78 (2H, d, *J* = 8 Hz), 7.82 (2H, d, *J* = 8 Hz), 7.89 (1H, d, *J* = 3.7 Hz). MS *m*/*z* 362 (M + H)⁺.

And Compound II:
¹H NMR (500 MHz, DMSO-d₆, δ, ppm): 2.93 (6H. s), 6.76 (2H, d, *J* = 9.2 Hz), 6.92 (1H, d, *J* = 3.8 Hz), 7.41 (1H, t, *J* = 7.6 Hz), 7.63-7.69 (4H, m), 7.79-7.82 (2H, m), 7.85 (1 H, d, *J* = 7.6 Hz). MS *m*/*z* 362 (M + H)⁺.

The reaction route for preparing the ¹⁰B-containing compound is shown in Reaction Formula II:

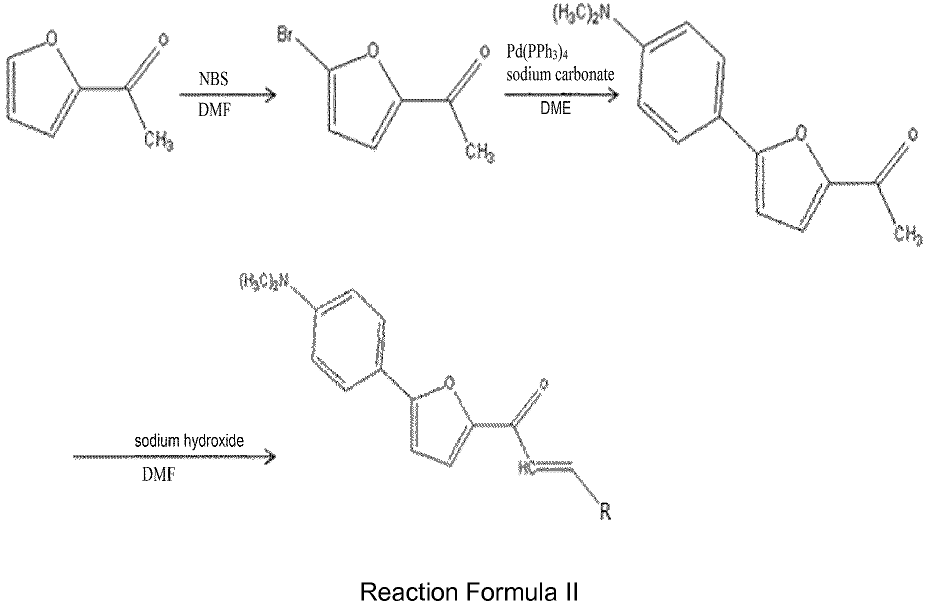

### Example 2. Use of ¹⁰B-containing compound in the preparation of a drug specifically bound to amyloid β-protein

Due to the presence of the blood-brain barrier (BBB), most compounds are difficult to enter the brain through the blood stream. For many drugs, they cannot exert their efficacy until passing through the blood-brain barrier. In general, water-soluble drugs are difficult to pass through the blood-brain barrier, and fat-soluble drugs have better permeability to water-soluble drugs. The dissolution, absorption, distribution, and transport of drugs in the body are related to the water-solubility and fat-solubility of drugs, i.e., the oil-water partition coefficient (logP). The oil-water partition coefficient is the logarithm of the partition coefficient ratio of the drug in the n-octanol and water phases. The greater the logP value is, the more lipophilic the substance is, otherwise it is easier to dissolve in water.

It has been reported that the value of the oil-water partition coefficient (logP) of the substance is preferably between 1 and 3. According to the experimental calculation in the report, the fat-water partition coefficient of the ¹⁰B-containing compound according to the example of the present disclosure is 2.97, and thus the ¹⁰B-containing compound has a good blood-brain barrier permeability when used for the preparation of a drug for eliminating amyloid β-protein deposition plaque.

In the examples of the present disclosure, the affinity of the ¹⁰B-containing compound and the amyloid β-protein deposition plaque is evaluated by using the equilibrium dissociation constant K_{D} value. K_{D} can indicate the degree of dissociation of the two substances in the equilibrium state. The larger the K_{D} value is, the more the dissociation is, indicating that the affinity between the two substances is weaker. The smaller the K_{D} value is, the less the dissociation is, indicating that the affinity between the two substances is stronger.

The amyloid β-protein solution was prepared at a concentration of 10 µM and mixed with compound I or compound II at different concentrations (concentrations ranging from 0.1 to 10 µM). After standing at room temperature for 20 min, the equilibrium dissociation constant K_{D} was measured and calculated. In addition, a known compound capable of specifically binding to amyloid β-protein was used as a control and mixed with a amyloid β-protein solution at a concentration of 10 µM and allowed to stand at room temperature for 20 min. The equilibrium dissociation constant K_{D} was also measured and calculated. The equilibrium dissociation constant of compound I is 0.79, the equilibrium dissociation constant of compound II is 0.9, and the equilibrium dissociation constant of the control is 1.59, thereby illustrating that Compound I and Compound II have stronger affinity with amyloid β-protein than the known compound capable of specifically binding to amyloid β-protein.

Wherein, the compound capable of specifically binding to amyloid β-protein in the control sample has the following structural formula:

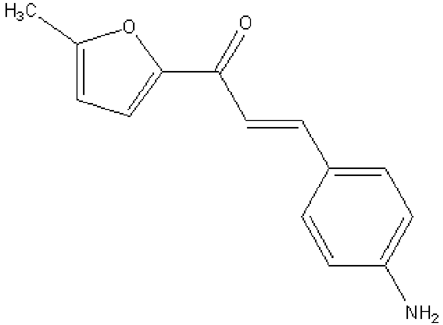

The drug produced by the ¹⁰B-containing compound of the present disclosure needs to pass through the blood-brain barrier to specifically bind to amyloid β-protein and be applied to the neutron capture therapy system to further eliminate the amyloid β-protein. From this example, it can be proved that the ¹⁰B-containing compound can be used to prepare a drug that specifically binds to amyloid β-protein and enable the drug to eliminate amyloid β-protein in a neutron capture therapy system.

### Example 3. Simulation test of neutron capture therapy system for eliminating amyloid β-protein deposition plaque

In this example, boronic acid (H₃¹⁰BO₃) was used in place of ¹⁰B-containing compounds (including compound I and compound II), wherein the boron element in boric acid (H₃¹⁰BO₃) was ¹⁰B, and bovine serum albumin (BSA) was used to mimic amyloid β-protein. The mixed solution of boric acid and bovine serum albumin was placed in a neutron beam environment generated by a neutron capture therapy device. The effect of neutron on bovine serum albumin and the effect of neutron beam on bovine serum albumin in the presence of H₃¹⁰BO₃ were analyzed by SDS-PAGE gel electrophoresis.

### (I) Effect of neutron on bovine serum albumin

A BSA solution with a concentration of 0.01% (w/w) was prepared with ultrapure water, and the prepared solution was stored and operated at 4°C. 1 mL of the BSA solution was placed on the centerline of the exit of the collimator of the neutron capture therapy device, wherein the distance of the solution from the exit of the collimator was 2cm and the neutron capture therapy device was set so that the neutron intensity at the exit of the collimator was 2.4×10¹¹/s, and the BSA solution was irradiated in the neutron environment for 2h; another 1mL of the BSA solution was taken as a control solution without neutron irradiation.

The BSA solution with 2h of neutron irradiation and the control solution were stained with Coomassie brilliant blue and subjected to SDS-PAGE gel electrophoresis, the colors of the protein bands in the electrophoresis patterns of the sample solution and the control solution were quantified by Image J software, and the values were used to represent the relative contents of proteins, wherein the content of BSA in the control solution was defined as 1. Under the above experimental conditions of neutron irradiation, the content of BSA after 2h of neutron irradiation was 0.8, and its content decreased by about 20%. It can be seen that the radiation containing a neutron beam can affect the protein content.

### (II) Effect of neutron on bovine serum albumin in the presence of H₃¹⁰BO₃

A solution of BSA and H₃¹⁰BO₃ was prepared with ultrapure water, wherein in the solution, the concentration of BSA was 0.01% (w/w), and the concentration of H₃¹⁰BO₃ was 0.18M; and the prepared solution was stored and operated at 4°C. 8 aliquots (numbered as A, B, C, D, E, F, G, H, respectively) were taken from the solution respectively, and 1mL of each solution was irradiated with the neutron capture therapy device. 8 aliquots of the solution were respectively placed on the center line of the exit of the collimator of the neutron capture therapy device, Solution A was 2cm from the exit of the collimator, Solution B was 4cm from the exit of the collimator, Solution C was 6cm from the exit of the collimator, and so on. The beam at the exit of the collimator included not only neutron ray, but also γ-rays and other radiations. In fact, it was mainly the neutron ray that had a destructive effect on proteins. In the example, the intensity of the beam was described as the neutron intensity in the beam, wherein, the neutron strength used in the present example was 2.4×10¹¹/s, and 8 aliquots of the solution were irradiated for 2h in the neutron environment; and another 1mL of the BSA and H₃¹⁰BO₃ solution was used as a control solution without neutron irradiation.

The control solution and the 8 aliquots of the solution irradiated by the radiation of the neutron capture therapy device were stained with Coomassie Brilliant Blue and subjected to SDS-PAGE gel electrophoresis. Figure 2 shows the SDS-PAGE electrophoresis pattern of the control solution and the 8 aliquots of the solution.

The first two protein bands in Figure 2 were BSA in the control solution and the rest were BSA after exposure to the radiation. 8 aliquots of the solution were placed on the center line of the exit of the collimator. Since all the solutions on the center line contained H₃¹⁰BO₃ and the ¹⁰B element has a large thermal neutron capture cross section, the neutron dose decreased significantly after the neutrons in the radiation from the exit of the collimator were passed through the solutions containing H₃¹⁰BO₃. The farther the solution was away from the collimator, the less radiation dose the BSA received.

As can be seen from Figure 2, the colors of the protein bands of the eight neutron-irradiated solutions became lighter in different degrees compared to that of the control. And, the closer to the exit of the collimator, the lighter the colors of the protein bands in the solutions were, indicating the more the protein content was reduced, and the closer to the exit of the collimator, the greater the neutron radiation doses received by the solution were. It was further illustrated that the amount of the neutron dose affected the content of BSA in the solution, and the larger the neutron dose was, the less the content of BSA in the solution after the neutron irradiation was.

The colors of the BSA protein bands in the electrophoresis patterns corresponding to the control solution and 8 aliquots of the solution were quantified by Image J software, and the values were used to represent the relative contents of the proteins, wherein the content of BSA in the control solution was defined as 1. Under the above experimental conditions of neutron irradiation, the contents of BSA after 2h of neutron irradiation are shown in Table 1.

It can be seen from Table 1, the content of BSA in the solutions irradiated by neutrons decreased to varying degrees. The solution placed 2cm away from the exit of the collimator was irradiated with neutrons with a neutron intensity of 2.4×10¹¹/s for 2h, leaving only 5.3% of its BSA content, indicating that the neutron can greatly destroy the structure of BSA and decrease the content of BSA in the presence of H₃¹⁰BO₃. And within the allowable range of experimental error, as the distances between the solutions and the exit of the collimator outlet became longer, generally the BSA contents of the eight solutions tended to decrease, further indicating that the amount of the neutron dose affected the BSA content.

**Table 1 Effect of neutron on bovine serum albumin in the presence of H₃¹⁰BO₃**

| **Solution number** | **BSA content (%)** |
|---|---|
| Control solution | 100 |
| A | 5.3 |
| B | 2.6 |
| C | 18.9 |
| D | 14.0 |
| E | 22.9 |
| F | 35.1 |
| G | 49.6 |
| H | 60.7 |

The compound I and the compound II provided by the present disclosure both carry a nuclide ¹⁰B with a large thermal neutron capture cross section as H₃¹⁰BO₃, and the compounds are capable of specifically binding to the amyloid β-protein. The compounds are placed in an environment containing amyloid β-protein, and the compounds will form a high concentration around the amyloid β-protein. Then the region where the compounds accumulate is irradiated with a neutron beam emitted from a neutron capture therapy device, and the energy released can destroy the structure of the amyloid β-protein. The ¹⁰B-containing compound according to the present disclosure is also fluorescent due to the nature of the molecule itself, so that the ¹⁰B-containing compound can also be used to detect or localize amyloid β-protein deposition plaque in vivo, in addition to eliminating amyloid β-protein deposition plaque in the neutron capture therapy system. Since the ¹⁰B-containing compound is fluorescent, in the process of eliminating amyloid β-protein deposition plaque, it is possible to determine the optimal timing of irradiation with a boron neutron capture therapy device by measuring its fluorescence intensity.

In summary, the ¹⁰B-containing compound has a strong blood-brain barrier penetrating ability and is capable of specifically bind to amyloid β-protein deposition plaque. And, since the ¹⁰B element in the ¹⁰B-containing compounds has a very high thermal neutron capture cross section, the ¹⁰B-containing compound can be used in a neutron capture therapy system to eliminate amyloid β-protein deposition plaque.

## Claims

1. A neutron capture therapy system for eliminating amyloid β-protein deposition plaque, comprising:
a neutron capture therapy device, and
a ¹⁰B-containing compound having a structure shown in structural formula I: 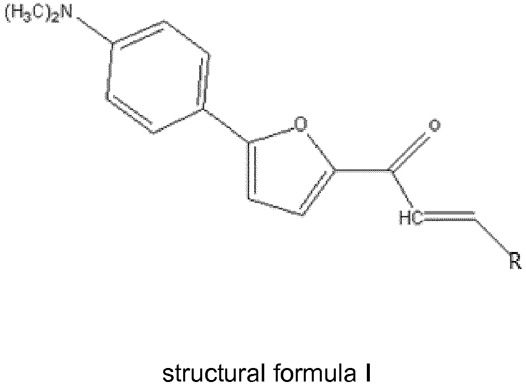
wherein, R is a phenylboronic acid group, and the boron in the phenylboronic acid group is ¹⁰B;
wherein the ¹⁰B-containing compound is capable of specifically binding to the amyloid β-protein deposition plaque, and the energy generated by action of a neutron beam generated by the neutron capture therapy device on the ¹⁰B-containing compound destroys the amyloid β-protein deposition plaque that is specifically bound to the ¹⁰B-containing compound.

2. The neutron capture therapy system for eliminating amyloid β-protein deposition plaque according to claim 1, wherein R group in the structural formula I is classified into R₁ and R₂ according to the substitution position of the boronic acid group, wherein R₁ represents: 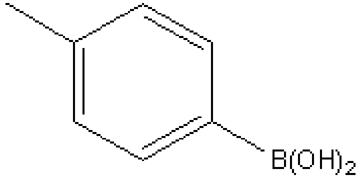 R₂ represents: 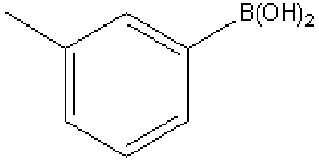 when the substituent R in the ¹⁰B-containing compound is R₁, the ¹⁰B-containing compound is Compound I; when the substituent R in the ¹⁰B-containing compound is R₂, the ¹⁰B-containing compound is Compound II.

3. The neutron capture therapy system for eliminating amyloid β-protein deposition plaque according to any of claims 1 or 2, wherein the neutron capture therapy device comprises:
a neutron source for generating a neutron beam,
a beam shaping assembly located at the rear of the neutron source, wherein the beam shaping assembly adjusts fast neutrons in the neutron beam having a wide energy spectrum generated by the neutron source to epithermal neutrons, and
a collimator for converging the epithermal neutrons.

4. The neutron capture therapy system for eliminating amyloid β-protein deposition plaque according to claim 3, wherein the neutron source comprises an accelerator-based neutron source or a reactor-based neutron source.

5. The neutron capture therapy system for eliminating amyloid β-protein deposition plaque according to claim 3, wherein the beam shaping assembly comprises:
a moderator for moderating fast neutrons into epithermal neutrons,
a reflector surrounding the moderator, wherein the reflector reflects neutrons diffused towards outside of the beam shaping assembly back into the moderator,
a thermal neutron absorber for absorbing thermal neutrons to avoid overdosing in superficial normal tissues during therapy, and
a radiation shield for shielding leaked neutrons and photons to reduce normal tissue dose in a non-irradiated area.

6. The neutron capture therapy system for eliminating amyloid β-protein deposition plaque according to any of claims 1 to 5, wherein the amyloid β-protein deposition plaque comprises Aβ₄₂.

## Patentansprüche

1. Neutroneneinfangtherapiesystem zum Eliminieren von Amyloid-ß-Protein-Ablagerungsplaque, umfassend:
eine Neutroneneinfangtherapievorrichtung, und
eine ¹⁰B enthaltende Verbindung, die die in der strukturellen Formel I gezeigte Struktur aufweist: 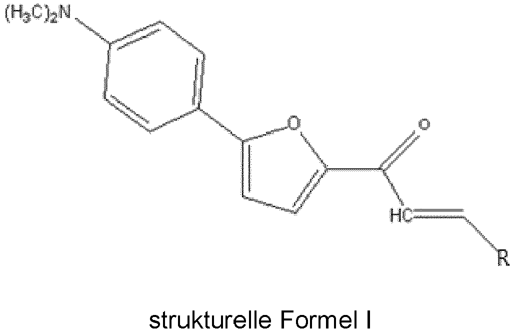
wobei R eine Phenylboronsäuregruppe ist und das Boron in der Phenylboronsäuregruppe ¹⁰B ist;
wobei die ¹⁰B enthaltende Gruppe in der Lage ist, sich spezifisch an die Amyloid-ß-Protein-Ablagerungsplaque zu binden und die Energie, die durch die Wirkung eines Neutronenstrahls erzeugt wird, der durch die Neutroneneinfangtherapievorrichtung erzeugt wird, auf die ¹⁰B enthaltende Verbindung die Amyloid-ß-Protein-Ablagerungsplaque, die spezifisch an die ¹⁰B enthaltende Verbindung gebunden ist, zerstört.

2. Neutroneneinfangtherapiesystem zum Eliminieren von Amyloid-ß-Protein-Ablagerungsplaque nach Anspruch 1, wobei die R-Gruppe in der strukturellen Formel I je nach der Substitutionsposition der Boronsäuregruppe in R₁ und R₂ eingeordnet ist, wobei R₁: 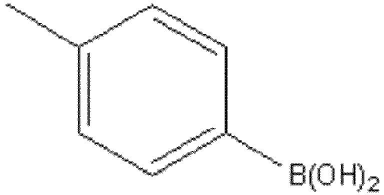 darstellt,
R₂: 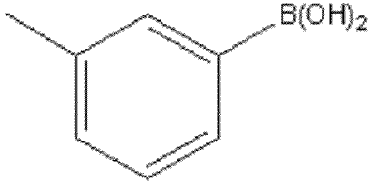 darstellt,
wenn der Substituent R in der ¹⁰B enthaltenden Verbindung R₁ ist, ist die ¹⁰B enthaltende Verbindung die Verbindung I; wenn der Substituent R in der ¹⁰B enthaltenden Verbindung R₂ ist, ist die ¹⁰B enthaltende Verbindung die Verbindung II.

3. Neutroneneinfangtherapiesystem zum Eliminieren von Amyloid-ß-Protein-Ablagerungsplaque nach einem der Ansprüche 1 oder 2, wobei die Neutroneneinfangtherapievorrichtung Folgendes umfasst:
eine Neutronenquelle zum Erzeugen eines Neutronenstrahls,
eine Strahlgestaltungsanordnung, die sich am Rücken der Neutronenquelle befindet, wobei die Strahlgestaltungsanordnung schnelle Neutronen in dem Neutronenstrahl, der ein breites Energiespektrum aufweist, das durch die Neutronenquelle erzeugt wird, zu epithermalen Neutronen einstellt, und
einen Kollimator zum Zusammenbringen der epithermalen Neutronen.

4. Neutroneneinfangtherapiesystem zum Eliminieren von Amyloid-ß-Protein-Ablagerungsplaque nach Anspruch 3, wobei die Neutronenquelle eine Neutronenquelle auf Beschleunigerbasis oder eine Neutronenquelle auf Reaktorbasis umfasst.

5. Neutroneneinfangtherapiesystem zum Eliminieren von Amyloid-ß-Protein-Ablagerungsplaque nach Anspruch 3, wobei die Strahlgestaltungsanordnung Folgendes umfasst:
einen Moderator zum Moderieren schneller Neutronen zu epithermalen Neutronen,
einen Reflektor, der den Moderator umgibt, wobei der Reflektor Neutronen, die auf die Außenseite der Strahlgestaltungsanordnung zu zerstreut worden sind, in den Moderator zurückwirft,
einen Absorber thermischer Neutronen zum Absorbieren thermischer Neutronen, um das Überdosieren in normalen Oberflächengeweben während der Therapie zu vermeiden, und
eine Strahlungsabschirmung zum Abschirmen entwichener Neutronen und Fotonen zum Reduzieren normaler Gewebedosis in einem nichtbestrahlten Bereich.

6. Neutroneneinfangtherapiesystem zum Eliminieren von Amyloid-ß-Protein-Ablagerungsplaque nach einem der Ansprüche 1 bis 5, wobei die Amyloid-ß-Protein-Ablagerungsplaque Aß₄₂ umfasst.

## Revendications

1. Système de thérapie par capture de neutrons permettant d'éliminer la plaque de dépôt de la béta-protéine amyloïde, comprenant :
un dispositif de thérapie par capture de neutrons, et
un composé contenant du ¹⁰B ayant une structure répondant à la formule structurelle I : 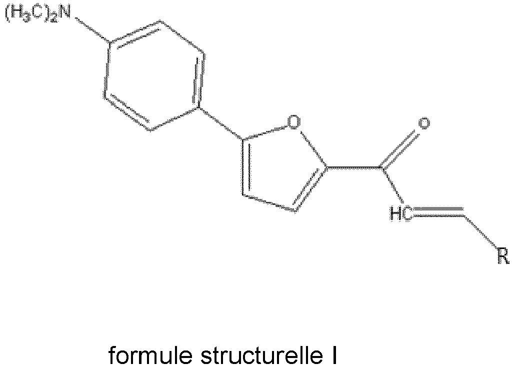
dans laquelle, R représente un groupe acide phénylboronique, et le bore dans le groupe acide phénylboronique est le ¹⁰B ;
dans laquelle le composé contenant du ¹⁰B est capable de se lier spécifiquement à la plaque de dépôt de la béta-protéine amyloïde, et l'énergie générée par l'action d'un faisceau de neutrons généré par le dispositif de thérapie par capture de neutrons sur le composé contenant du ¹⁰B détruit la plaque de dépôt de la béta-protéine amyloïde qui est spécifiquement liée au composé contenant du ¹⁰B.

2. Système de thérapie par capture de neutrons permettant d'éliminer la plaque de dépôt de la béta-protéine amyloïde selon la revendication 1, dans lequel le groupe R dans la formule structurelle I est classé en R₁ et R₂ conformément à la position de substitution du groupe acide phénylboronique, dans lequel
R₁ représente : 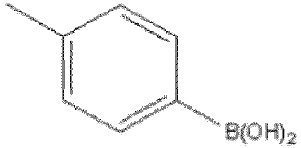 R2 représente : 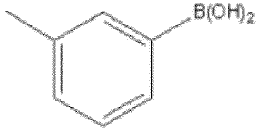 lorsque le substituant R dans le composé contenant du ¹⁰B est R₁, le composé contenant du ¹⁰B est le composé I ; lorsque le substituant R dans le composé contenant du ¹⁰B est R₂, le composé contenant du ¹⁰B est le composé II.

3. Système de thérapie par capture de neutrons permettant d'éliminer la plaque de dépôt de la béta-protéine amyloïde selon la revendication 1 ou la revendication 2, dans lequel le dispositif de thérapie par capture de neutrons comprend :
une source de neutrons permettant de générer un faisceau de neutrons,
un ensemble de mise en forme de faisceau situé à l'arrière de la source de neutrons, dans lequel l'ensemble de mise en forme de faisceau règle les neutrons rapides dans le faisceau de neutrons ayant un large spectre d'énergie généré au moyen de la source de neutrons en des neutrons épithermiques, et
un collimateur permettant de converger les neutrons épithermiques.

4. Système de thérapie par capture de neutrons permettant d'éliminer la plaque de dépôt de la béta-protéine amyloïde selon la revendication 3, dans lequel la source de neutrons comprend une source de neutrons basée sur un accélérateur ou une source de neutrons basée sur un réacteur.

5. Système de thérapie par capture de neutrons permettant d'éliminer la plaque de dépôt de la béta-protéine amyloïde selon la revendication 3, dans lequel l'ensemble de mise en forme de faisceau comprend :
un modérateur permettant de modérer les neutrons rapides en des neutrons épithermiques,
un réflecteur entourant le modérateur, dans lequel le réflecteur réfléchit les neutrons diffusés en direction de l'extérieur de l'ensemble de mise en forme de faisceau de retour vers le modérateur,
un absorbeur de neutron thermique permettant d'absorber les neutrons thermiques afin d'éviter le surdosage dans les tissus normaux superficiels pendant la thérapie, et
un écran de protection radiologique permettant de se protéger des neutrons et des photons qui ont fui afin de réduire le dosage de tissu normal dans une zone non irradiée.

6. Système de thérapie par capture de neutrons permettant d'éliminer la plaque de dépôt de la béta-protéine amyloïde selon l'une quelconque des revendications 1 à 5, dans lequel la plaque de dépôt de la béta-protéine amyloïde comprend le Aβ₄₂.
